(19) Europäisches Patentamt / European Patent Office / Office européen des brevets

(11) **EP 3 086 118 A1**

(12) **EUROPEAN PATENT APPLICATION**

(43) Date of publication:
26.10.2016 Bulletin 2016/43

(51) Int Cl.:
*G01N 33/06* (2006.01)   *G01N 22/00* (2006.01)

(21) Application number: 16166198.8

(22) Date of filing: 20.04.2016

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 20.04.2015 US 201562149866 P

(71) Applicant: Vayyar Imaging Ltd.
5610103 Yehud (IL)

(72) Inventor: SHAYOVITZ, Shachar
Ness Ziona (IL)

(74) Representative: Modiano, Gabriella Diana et al
Modiano & Partners (DE)
Thierschstrasse 11
80538 München (DE)

(54) **TEMPERATURE COMPENSATED DIELECTRIC CHARACTERIZATION OF SUBSTANCES**

(57)   A system device and methods for temperature compensation for RF systems and devices such as devices for dielectric characterizing of one or more substances where the substances have temperature-dependent behavior. Such system can be used for radio frequency dielectric monitoring of foodstuffs. The system (100) comprises RF sensors (130, 135) and a processor (160) for analyzing the substances according to impedance or dielectric properties measures of the substances.

Figure 1

**Description**

**INCORPORATION BY REFERENCE**

All publications, patents, and patent applications mentioned in this specification are herein incorporated by reference to the same extent as if each individual publication, patent, or patent application was specifically and individually indicated to be incorporated by reference.

**FIELD OF THE INVENTION**

The present invention relates to a sensing system device and method for characterizing an object or substances and more specifically, but not exclusively, to temperature compensated characterization of nonsolid object such as liquid or ointment using Radio Frequency (RF) sensors such as microwave sensors.

**BACKGROUND**

From time immemorial there was a need to recognize and diagnose substances, objects, or samples including complex mixtures, e.g. foodstuffs to obtain the ingredients of the sample or to identify whether the substances have been tampered with or adulterated. Immediate and precise identification of objects under examination may include recognizing the ingredients of the substances such as a mixture or to identify defects, forgery or diseases in substances.
Temperature variation is one of the most significant sources of gaging error while examining the substances. An analysis of substances measured in different temperature conditions such as varied ambient temperature will result in imprecise measurement and sometime in an incorrect measurement result. Specifically, some substances such as liquids are more sensitive to temperature variations than solid substances.

**SUMMARY OF INVENTION**

It is an object of the present invention to provide a system, device and methods for characterizing an object of substances such as temperature dependence substances for example non-solid substances and obtain information on the ingredients of the substances while compensating the temperature effect on the characterization process.
It is yet another object of the present invention to provide a system and methods that will allow obtaining information, for example but not limited to, testing of an object such as milk, ink or drug to see if it has been adulterated.
It is an object of the present invention to provide a system, device and methods for characterizing substances such as non-solid substances wherein the substances are not in direct contact with the system sensing equipment.
According to a first aspect of the invention there is provided a system for characterizing a substance, said system comprising: a housing body having a cavity therein wherein said cavity is configured to contain said substance; a transmit unit configured to transmit a plurality of Radio Frequency (RF) signals towards said substance; at least one electromagnetic sensor, wherein said at least one electromagnetic sensor is configured to receive RF signals affected by said substance and provide RF responses data of said substance; a Radio Frequency Signals Measurement Unit (RFSMU) configured to receive said RF responses data and measure said RF responses data; a temperature sensor configured to measure the temperatures of said substance over time; and at least one processing unit, said at least one processing unit is configured to: retrieve temperature dependent RF response model of said substance; receive the temperatures and the measured RF responses data of said substance; normalize the measured RF responses of said substance according to said temperature dependent RF response model to generate compensated measured RF responses of said substance at a reference temperature; and process said compensated measured RF responses to identify the characteristics of said substance.
In an embodiment, said characteristics are dielectric properties of said substance.
In an embodiment, said temperature dependent RF response model is configured by measuring said RF responses data for several values of temperatures and fitting said model.
In an embodiment, said substance is a mixture of materials and the characteristics are percentage values of the mixture of materials.
In an embodiment, the identification of characteristics of said substance is performed using a model generated by measuring said compensated RF responses for several values of said characteristics and fitting said model.
In an embodiment, the temperatures are obtained by the at least one electromagnetic sensor.
In an embodiment, said at least one electromagnetic sensor comprises at least two antennas and wherein said at least one processing unit is configured to analyze a passage of energy transmitted from at least one of said antennas to at least one other antenna.
In an embodiment, said at least one electromagnetic sensor is a capacitive sensor and wherein said sensor is configured to spatially resolve the dielectric properties of the said substance.

In an embodiment, said one electromagnetic sensor is attached internally or externally to the housing body.

In an embodiment, said at least one electromagnetic sensor is connected to said housing body and said transmit unit.

In an embodiment, said at least one electromagnetic sensor in not in direct contact with said substance.

In an embodiment, said substance is a non-solid substance.

In an embodiment, said substance is in the form selected from a group consisting of: liquid, paste, gas, granular material.

In an embodiment, said liquid is selected from the group consisting of: oil, milk, ink, water, bodily fluids, liquid mixture.

In an embodiment, said substance is an agricultural product.

In an embodiment, the dielectric properties of the substance are configured based on an estimation model, said estimation model is based on measuring RF signals affected by plurality of substances, wherein said plurality of substances parameters are known.

According to a second aspect of the invention there is provided a method for characterizing a substance, the methods comprising: transmitting a plurality of Radio Frequency (RF) signals from a transmit unit towards said substance; receiving said transmitted RF signals by at least one electromagnetic sensor;

providing RF responses data of said substance by said at least one electromagnetic sensor based on said plurality of RF signals; obtaining the plurality RF signals by a Radio Frequency Signal Measurement Unit (RFSMU); measuring the obtained RF signals by the RFSMU; measuring the temperatures of said substance over time to yield temperature data of said substance; retrieving a temperature dependent RF response model of said substance by at least one processing unit; normalizing the RF responses data of said substance according to said temperature dependent RF response model; generating compensated RF responses of said substance at a reference temperature; and processing said compensated RF responses to identify the characteristics of said substance.

In an embodiment, said characteristics are dielectric properties of said substance.

In an embodiment, said temperature dependent RF response model is configured by measuring said RF responses data for several values of temperature and fitting said model.

In an embodiment, said substance is a mixture of materials and the characteristics are a percentage values of the materials.

In an embodiment, the identification of characteristics of said substance is performed using a model generated by measuring said compensated RF responses for several values of said characteristics and fitting said model.

In an embodiment, the temperatures are obtained by the at least one electromagnetic sensor.

In an embodiment, said at least one electromagnetic sensor comprises at least two antennas and wherein said processor is configured to analyze a passage of energy transmitted from at least one of said antennas to at least one other antenna.

In an embodiment, said at least one electromagnetic sensor is a capacitive sensor and wherein said capacitive sensor is configured to spatially resolve the dielectric properties of the said substance.

In an embodiment, said at least one electromagnetic sensor is attached to a housing body or to a cavity of said housing and wherein said cavity is configured to contain said substance.

In an embodiment, said one electromagnetic sensor is attached internally or externally to the housing body.

In an embodiment, said temperature dependent RF response model comprises temperature dependence parameters, frequency dependence functions and temperature dependence functions

where the temperature dependent RF response model parameters are $a_0$ till $a_k$, $b_0$ till $b_k$, $c_0$ till $c_k$ and $d_0$ till $d_k$ and $\Delta T = (T_1 - T_0)$

$$\angle H_{T_1}(f) = \angle H_{T_0}(f) + (c_k \Delta T^k + c_{k-1} \Delta T^{k-1} + \cdots + c_0)(a_n f^n + a_{n-1} f^{n-1} + \cdots + a_1 f + a_0)$$

$$20\log_{10}|H_{T_1}(f)| = 20\log_{10}|H_{T_0}(f)| + (d_r \Delta T^r + d_{r-1} \Delta T^{r-1} + \cdots + d_0)(b_m f^m + b_{m-1} f^{m-1} + \cdots + b_1 f + b_0)$$

In an embodiment, $T_1$ and $T_0$ are the temperatures for the frequency responses $H_{T_1}(f)$ and $H_{T0}(f)$.

In an embodiment, said frequency dependence functions comprise polynomials $g_{phase}(f)$ and $g_{amp}(f)$.

In an embodiment, said temperature-dependence functions comprise polynomials $h_{amp}(T)$ and $h_{phase}(T)$ of a temperature difference $T_1 - T_0$.

In an embodiment, said temperature data comprises at least two temperature measurements over time of said substance.

In an embodiment, said temperature data comprises a desired reference temperature $T_0$.

In an embodiment, said temperature-dependence function comprises:

$$\angle H_{T_1}(f) = \angle H_{T_0}(f) + h_{phase}(T_1 - T_0)g_{phase}(f) \quad \text{and}$$

$$20\log_{10}|H_{T_1}(f)| = 20\log_{10}|H_{T_0}(f)| + h_{amp}(T_1 - T_0)g_{amp}(f)$$

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks, according to embodiments of the invention, could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein, are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

## BRIEF DESCRIPTION OF THE DRAWINGS

The subject matter disclosed may best be understood by reference to the following detailed description when read with the accompanying drawings in which:

Figure 1 is schematic view of a testing system including an antenna attached to or mounted on a container, according to an embodiment of the invention;

Figures 2A and 2B depict the testing system including a capacitive sensor, according to an embodiment of the invention;

Figure 3 depicts the testing system, according to another embodiment of the invention;

Figure 4 shows a flowchart of a method for characterizing one or more substances, wherein said substances characteristics are affected by temperature and compensating the temperature, according to an embodiment of the invention;

Figure 5 shows a flowchart of a method for providing a temperature dependent RF (Radio Frequency) response model, according to an embodiment of the invention;

Figure 6 shows a flowchart of a method for providing an estimation model for characterizing a substance, according to an embodiment of the invention;

Figure 7 shows a flowchart of a method for characterizing a substance where the properties of the substance are unknown, according to an embodiment of the invention;

Figure 8A, depicts measurement results of a substance where a calibration process is not performed, according to an embodiment of the invention; and

Figure 8B, depicts measurement results of a substance where a calibration process is performed according to an embodiment of the invention.

**DETAILED DESCRIPTION OF THE INVENTION**

The present invention relates to a sensing system, device and methods for characterizing an object or one or more substances and more specifically, but not exclusively, to system device and methods for temperature compensation for RF systems and devices such as devices for dielectric characterizing and analyzing of substances with temperature-dependent behavior according to impedance or dielectric properties measures of the substances using, for example RF sensors such as microwave sensors.

The embodiments disclosed herein can be combined in one or more of many ways to provide improved substances impedance measuring methods and apparatus. One or more components of the embodiments disclosed herein can be combined with each other in many ways.

A 'temperature compensation' according to the present invention is defined as a process of providing a device or system or methods independent of changes in temperature such as changes in ambient temperature or changes in the temperature of the object or substance under test.

According to one embodiments of the invention there is provided a system for characterizing a substance, said system comprising: a housing body having a cavity therein wherein said cavity is configured to contain said substance; a transmit unit configured to transmit a plurality of Radio Frequency (RF) signals towards said substance; at least one electromagnetic sensor, wherein said at least one electromagnetic sensor is configured to receive RF signals affected by said substance and provide RF responses data of said substance; a Radio Frequency Signals Measurement Unit (RFSMU) configured to receive said RF responses data and measure said RF responses data; a temperature sensor configured to measure the temperatures of said substance over time; and at least one processing unit, said at least one processing unit is configured to: retrieve temperature dependent RF response model of said substance; receive the temperatures and the measured RF responses data of said substance; normalize the measured RF responses of said substance according to said temperature dependent RF response model to generate compensated measured RF responses of said substance at a reference temperature; and process said compensated measured RF responses to identify the characteristics of said substance.

**Impedance Measurement Systems**

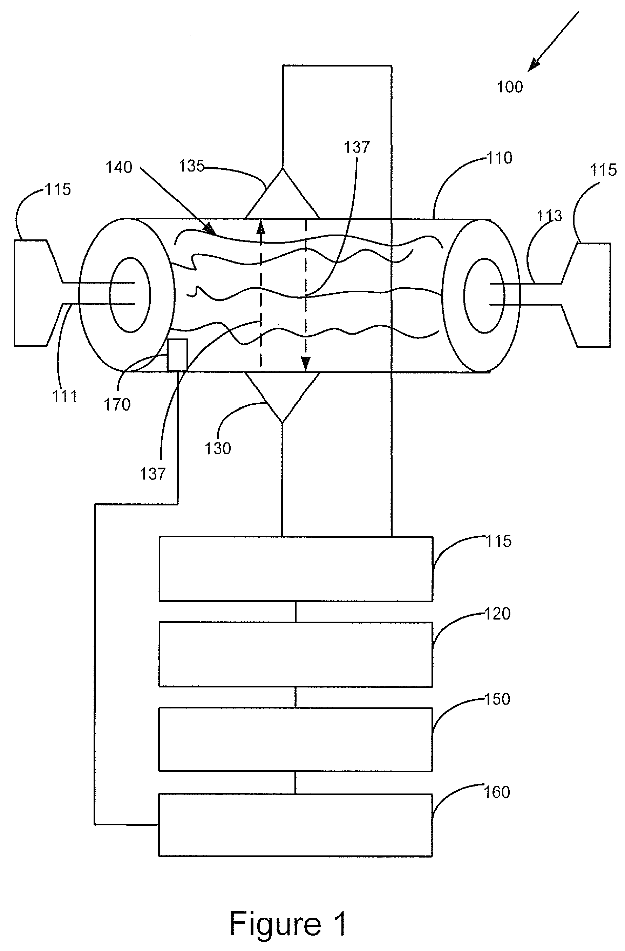

Referring now to the drawings, **Figure 1** illustrates a testing system **100** configured to characterize a sample material, e.g. obtain information on one or more objects, substances, or materials under test (hereinafter object(s) OUT or MUT or sample(s) or material(s) or substance(s)) **140** placed for example inside a housing **110** to identify the OUT ingredients, content or any change in the ingredients of the OUT. For example the OUT may be in the form of liquid or ointment placed in a housing such as a bottle or vessel and the testing system **100** may identify the components (e.g. the type and amount) found within the OUT. The results identified by the testing system **100** may be transmitted, displayed or shared to one or more users, for example in real-time, through various media, such as mobile telephone, internet or close loop TV system.

According to one embodiment of the invention, the system **100** may include a container, housing or vessel 110, whose diameter may be for example between 2-10cm, for example 5cm, holding the OUT **140**. The container may be in the shape of a pipe or any shape capable of holding the OUT **140**. The housing **110** may be connected to an OUT source **115** via one or more inlet and outlet pipes **111** and **113**. For example, the container may be part of or may be connected via the inlet pipe to a milking system and the OUT may be milk received directly from the milking system. The testing system **110** may include one or more microwave sensors (e.g. at least one electromagnetic sensor, configured to receive RF signals affected by the sensed substance and provide RF responses data of the substance) such as antenna sensors **135** and **130** attached to or located in proximity to the container **110**. The sensors **135** and **130** may transfer a plurality of RF signals **137** propagating a wave into the housing **110** for characterizing the OUT. The system **100** further includes a transmit/receive subsystem (e.g. transmit unit) **115** configured to generate and transmit the RF signals, for example, from 10MHz to 10GHz, to a Radio Frequency Signals Measurement Unit (RFSMU) **120** such as a Vector Network Analyzer (VNA) for measuring the received/reflected signals, a data acquisition subsystem **150** and further a processor unit **160** (e.g. at least one electronic processor unit) for processing the measured signals and characterising the OUT.

According to one embodiment of the invention as shown in **Figure 1** sensors **135** and **130** are externally connected to the housing **110** walls to generate a plurality of signals **137** penetrating the container walls from both sides and examining the OUT. Thus, enabling a sterilized measuring process and preventing the direct contact between the OUT and the antenna, and avoiding unwanted external substances such as dirt to influence the measurement process.

In one embodiment, the sensors such as sensors **135** and **130** may be multilayer structure implemented at least in part with printed circuit board techniques using appropriate dielectric materials. Commonly used materials are glass-epoxy, Teflon-based materials. Layers of high-dielectric-constant materials can be incorporated in order to match the antennas to materials under test.

According to one embodiment of the invention, the sensors **135** and **130** may include one or more antennas such as antenna array. The antennas can be of many types known in the art, such as printed antennas, waveguide antennas, dipole antennas or "Vivaldi" broadband antennas. The antenna array can be linear or two-dimensional, flat or conformal to the region of interest.

In some cases, the sensors are placed inside the container **110**, for example attached to the container inner walls or to a dedicated holder configured to hold the sensors in the container **110**.

The transmit/receive subsystem **115** is responsible for generation of the RF signals, coupling them to the antennas, reception of the RF signals from the antennas and converting them into a form suitable for acquisition. The signals can be pulse signals, stepped-frequency signals, chirp signals and the like. The generation circuitry can involve oscillators, synthesizers, mixers, or it can be based on pulse oriented circuits such as logic gates or step-recovery diodes. The conversion process can include down conversion, sampling, and the like. The conversion process typically includes averaging in the form of low-pass filtering, to improve the signal-to-noise ratios and to allow for lower sampling rates.

According to some embodiments of the invention, the transmit/receive subsystem **115** may perform transmission and reception with multiple antennas at a time or select one transmit and one receive antenna at a time, according to a tradeoff between complexity and acquisition time.

The data acquisition subsystem **150** collects and digitizes the signals from the transmit/receive subsystem **115** while tagging the signals according to the antenna combination used and the time at which the signals were collected. The data acquisition subsystem **150** will typically include analog-to-digital (A/D) converters and data buffers, but it may include additional functions such as signal averaging, correlation of waveforms with templates or converting signals between frequency and time domain.

The processing unit **160** (e.g. at least one electronic processor unit) is responsible for converting the collected signals into responses characterizing the OUT, and converting the sets of responses, into data relating to the OUT characteristics and compensating the temperature as will be described in details hereinbelow. The processing unit **160** is usually implemented as a high-performance computing platform, based either on dedicated Digital Signal Processing (DSP) units, general purpose CPUs, or, according to newer trends, Graphical Processing Units (GPU).

In some cases, the system may include one or more temperature sensors, such as thermostat **170**, located for example inside the container **110** or in contact with OUT **140**. The temperature sensors are configured to measure the OUT temperature over time for example several times e.g. every second or two seconds and provide a temperature data to processing unit **160**.

In some cases the temperature sensors are activated automatically, for example by the processing unit or by other units, once the sensors (e.g. sensors **130** and **135**) are activated as part of the OUT characterization process.

According to other embodiments, the system **100** doesn't include a dedicated temperature sensor and the OUT temperature may be obtained by the system's RF sensors such as sensors **130** and **135** according to methods as known in the art.

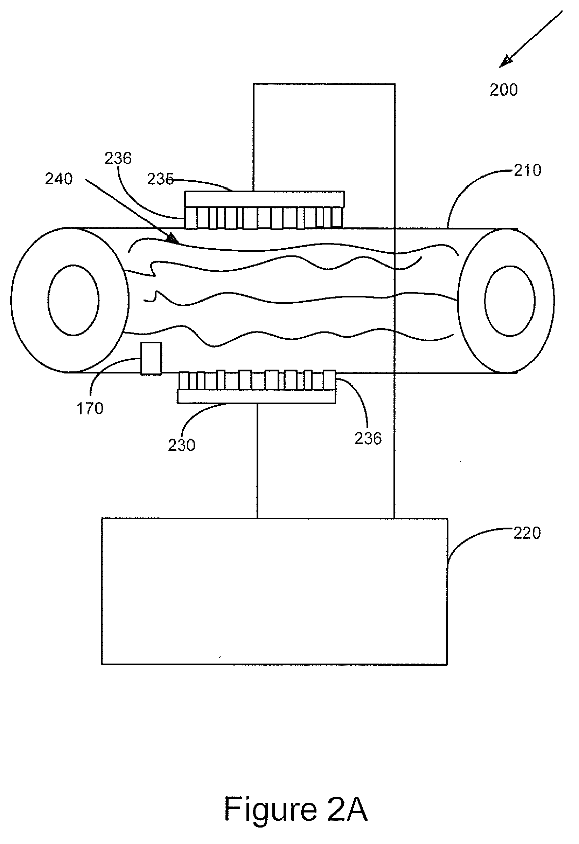

Figure 2A illustrates another embodiment of a testing system **200**. The testing system **200** includes a container such as a pipe **210** for holding the OUT **240**. A number of capacitive sensors such as two capacitive sensors **230** and **235** are connected to the container wall at multiple locations for spatially resolving the dielectric properties of the OUT. For example the capacitive sensor **235** is externally or internally connected to one side of the pipe **210** while capacitive sensor **230** is externally or internally connected to the other side of the pipe **210**.

According to one embodiment, the capacitive sensors **235** and **230** may include a number of sensing lines such as feed lines **236** forming a mutual capacitance meter on the pipe. The feed lines **236** may be organized in a structure of but not limited to a grid. Additionally, the capacitive sensors **235** and **230** may include a plurality of sensing plates at the surface of the sensor, connected to the feed lines **236**. Each of the feed lines is coupled to one or more corresponding receive lines through an electromagnetic field between at least two plates connected to said lines and to the OUT **240** located within the formed electromagnetic field in the pipe **210**. In one embodiment, the sensors **235** and **230** may include a number of layers, where the feed lines **236**, such as the column and row feed lines are placed in separate layers, and are connected to the sensing plates through vertical metalized holes (e.g. vias) interconnecting the layers. As shown in Figure 2A the capacitive sensors **235** and **230** may be connected to the transmit/receive subsystem **220** for generation of signals to the capacitive sensors.

In some cases the sensors are configured to measure the OUT complex impedance at several sampled frequencies over an excitation range from a few Hz to several GHz. Typically, the sensors may be operated over a number of frequencies between 10 and 50, advantageously over about thirty frequencies. For example, collecting 201 evenly spaced frequencies between 1GHz to 4GHz.

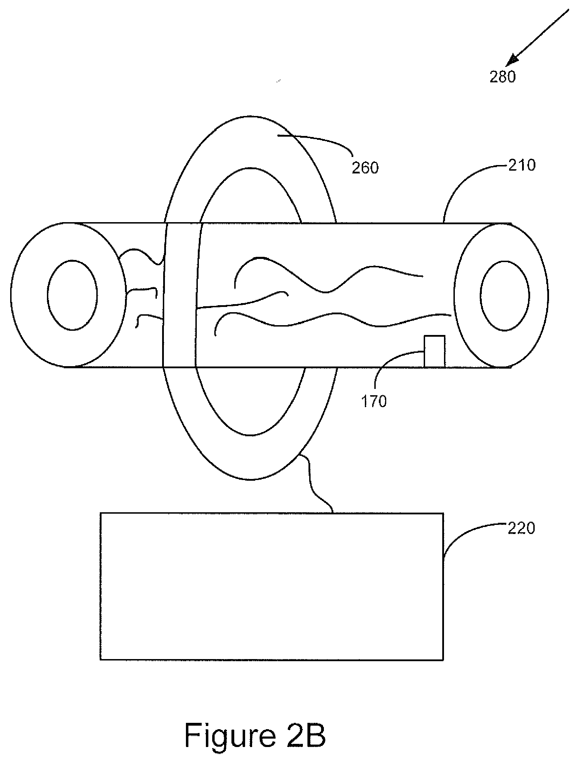

Figure 2B illustrates another embodiment of a testing device **280**. The testing device **280** may include a "ring shaped" capacitive sensor **260** surrounding the pipe **210**. For example the capacitive may partially or completely surround the outside or inside walls of the pipe **210**.

Examples for embodiments for characterizing a substance properties may be found in PCT Patent Application No. PCT/IL2015/050126, entitled "System device and method for testing an object" and US patent application publication number 2006/0255276 entitled "Device for analyzing the composition of the contents of a container" which application is incorporated by reference herein in its entirety.

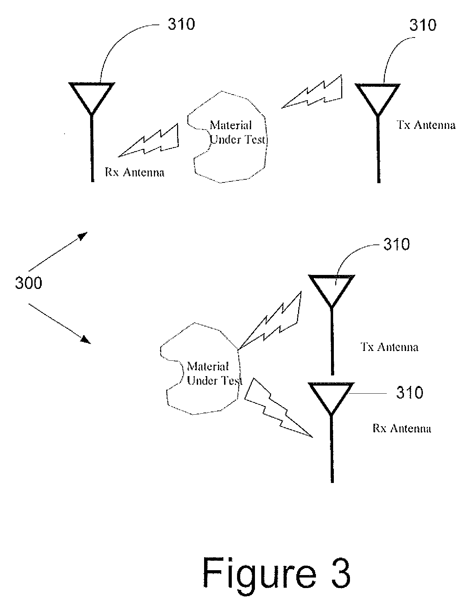

**Figure 3** shows a system **300** for characterizing one or more substances (e.g. MUT or OUT) comprising one or more transmission and reception antennas **310** configured to measure the properties of substances by transmitting an EM wave by the antennas **310** to measure the thru signal and/or the reflection signal from the substances. The substances may be for example liquid such as milk or liquid mixture such as Gasoline, fruit mixture, oil and may be located in proximity to the antennas for example few cm from the antennas, for example less than 50 cm, specifically 2cm. In some cases the substances may be placed or covered in a box or bottle or placed within a container.

In operation, the measurement process may include receiving by the antennas **310** the frequency response of an EM wave travelling through the substances and analyzing the impedance (e.g. phase and amplitude) of the received EM wave by a processor, such as one or more processing units or devices. The measurement process further includes providing data on the substances such characterising the substances or identifying the substances ingredients based on the analyzed amplitude and phase. In some cases the measured impedance is compared with predetermined reference such as database comprising impedance references. The processor unit may be for example the processor shown in Figure 1.

In some cases the substance is milk and the processing unit may analyze the received signals and measure the amplitude and phase of the signals passing through the milk to identify the dielectric properties of the milk. The amplitude and phase of the signals are indicative of the fat and protein level in the milk, and the level of milk's lactose.

For example, online milk analyzers in farms estimate certain properties of milk such as: fat, protein, lactose, somatic cell count and urea. The fat percentage of the milk is primarily related to the value of the dielectric constant and thus proportional to the phase response. It is stressed that other properties of the milk may be identified in accordance with embodiments of the invention.

## Temperature Compensation

As part of a measuring process of sample(s) such as non-solid substance(s) e.g., liquid, (milk or liquid mixtures) or solid sample(s) to characterize the sample(s) properties, the sample's temperature or ambient temperature may alter the spectral impedance response resulting in inaccurate and sometime in error estimation of the sample properties. For example, processing a plurality of RF signals obtained from the same sample along several seconds to measure the dielectric property of the sample may result in various dielectric results as if a number of samples were measured due to temperature variations of the same sample.

Specifically, during the measurement of the frequency response of an EM (electromagnetic) wave propagating through the milk, the temperature of the milk sample typically varies in time, and as a result the phase and amplitude response will vary regardless of the fat/protein/lactose percentages, causing an error or inaccuracy in estimation. Therefore, temperature-induced change may obscure the change induced by the change in material properties to be characterized. In other words, a temperature change, such as a change in the substance temperature or ambient temperature between measurements of the same substance may result in a false measurement.

According to one embodiment of the present invention there are provided systems and methods for characterizing an OUT properties comprising correcting or compensating the temperature effect by transforming (e.g. normalizing) the OUT's measurement results to equivalent results at a reference temperature value. The reference temperature is a temperature to which all the measurements are transformed as if they were measured at that temperature. This transformation (e.g. normalization) further eliminates the phase/amplitude drift resulted due to temperature effects which are not related to the properties of the OUT being estimated.

In some cases, the temperature effect is compensated by operating a temperature normalization manipulation process on the measured substance response, before estimating the substance properties.

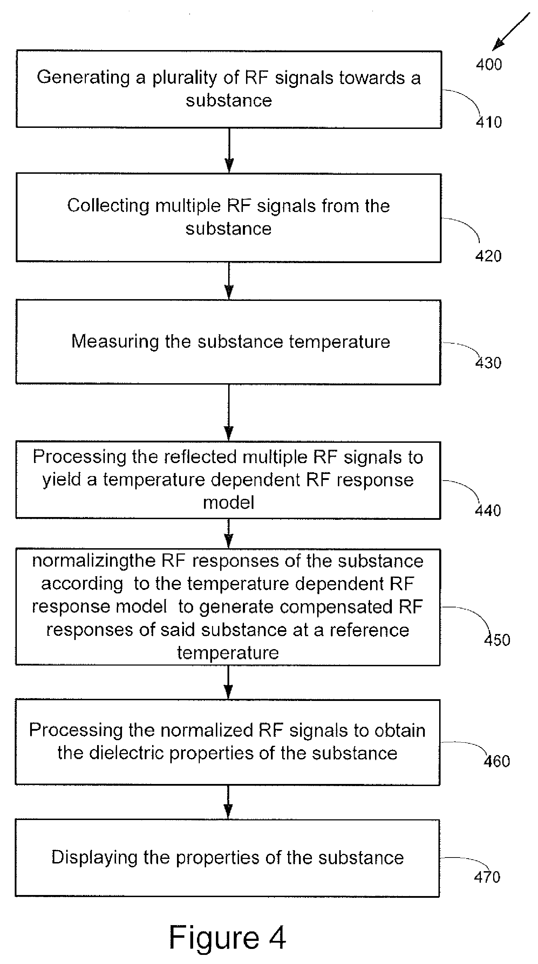

**FIG. 4** shows a flowchart **400** of a method for characterizing one or more substance(s), wherein said substance(s) characteristics are affected by temperature (e.g. temperature dependence substance(s)), and compensating the temperature, according to an embodiment of the invention. The method begins in step **410** which includes generating a plurality of RF signals, for example between 2GHz and 9GHz, by a plurality of RF sensors (e.g. antennas), such as the sensors attached to or placed in proximity to a container or sack comprising the substance(s) (e.g. OUT). The RF sensors may be any of the mentioned above sensors.

Step **420** includes collecting by the RF sensors multiple RF signals reflected from the substances and step **430** includes measuring the substance(s) (e.g. OUT) temperature to obtain temperature data of the OUT.

The temperature data may be obtained by any device or system as known in the art such as the thermometer **170** shown in Figures 1 and 2. Alternatively the temperature may be obtained by processing the RF signals by a processor unit as described above.

Step **440** includes processing reflected multiple RF signals and the temperate data of the OUT to yield a temperature dependent RF response model of the OUT (e.g. temperature frequency and phase dependent model $H_T(f)$).

To accurately and correctly characterize the substance(s) and measure the different properties of the substance(s) the processing step includes a calibration step e.g., calibrating the measured dielectric properties of the substance(s) to compensate the unwanted temperature effect on the measurement process. The calibration process includes normalizing the measured amplitude and phase of the substances according to the measured temperature dependent RF response model by transforming the measurement to a reference temperature that will eliminate the phase/amplitude drift on the substance(s). Therefore, step **450** includes normalizing the RF responses of the substance(s) according to the temperature dependent RF response model to generate compensated RF responses of said substance(s) at a reference temperature.

Step **460** includes processing the normalized RF signals to characterize the substance(s), e.g. measure the dielectric properties of the substance(s) and identify the quantitative qualities of the substances. The processing step may be activated for example by the processor unit and the Radio Frequency Signals Measurement Unit (RFSMU) connected to or in communication with the sensors as shown in Figures 1 -3.

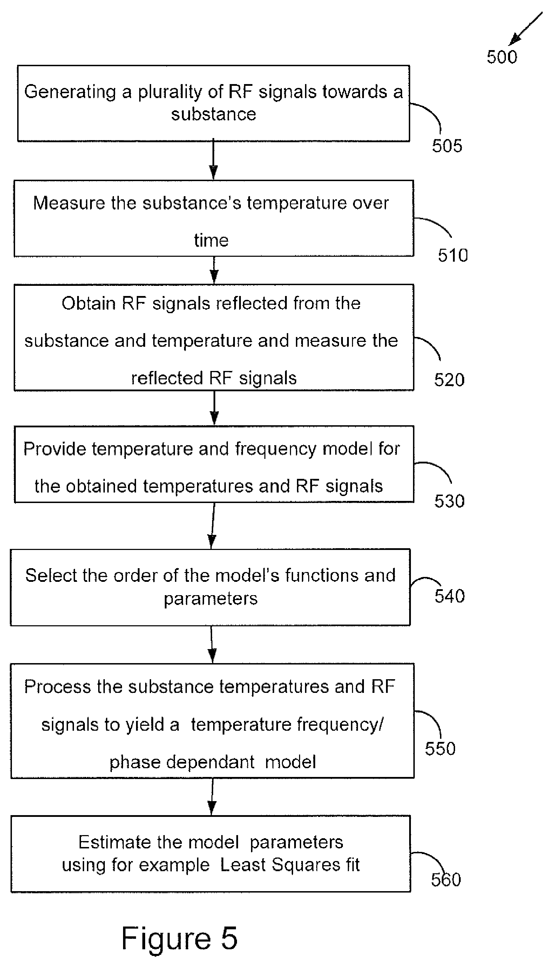

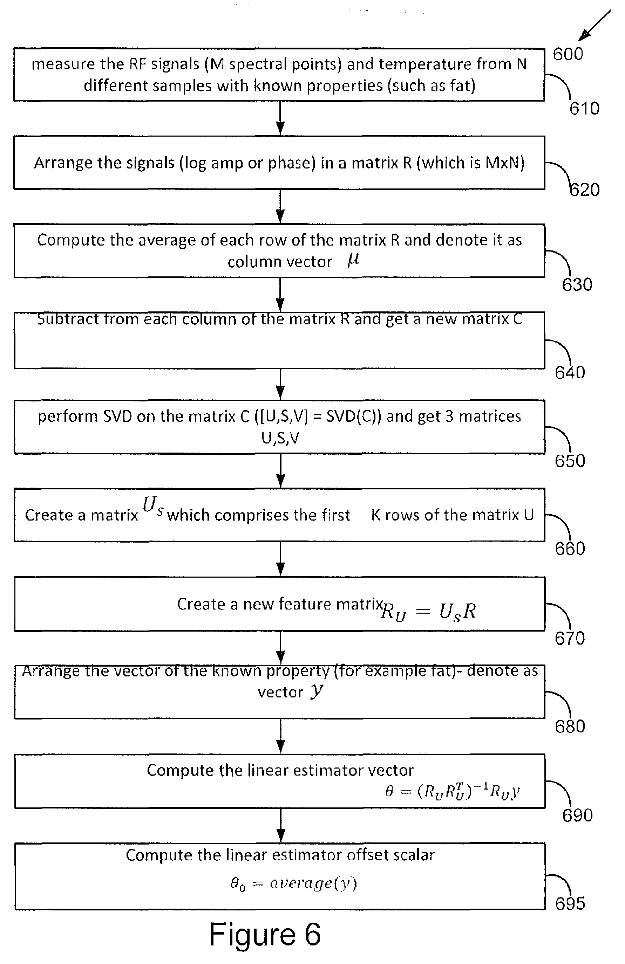

It is stressed that in some cases the referenced temperature model is obtained as part of the processing step (e.g. step **440**) as will be illustrated in Figures 5 and 6. Alternatively the referenced temperature model may be obtained separately according to previous measurements processed on other referenced substances and implemented automatically by the processor unit.

Step **470** includes displaying the results of the measurement for example presenting details on a milk content, such as fat or lactose percentage.

The steps of the method of **Figure 4** (and its various alternatives) may be embodied in hardware or software including a non-transitory computer-readable storage medium (e.g., an optical disc or memory card) having instructions executable by a processor of a computing device.

**Referenced Temperature Model**

In accordance with embodiments of the invention there is provided a method for temperature dependence estimation which provides an estimating of the temperature dependence functions defined as: $h_{amp}(T)$ and $h_{phase}(T)$ and the frequency dependence functions defined as: $g_{phase}(f)$ and $g_{amp}(f)$. Following a collecting phase which includes obtaining several RF responses of the same OUT at different temperatures, the model is estimated. The resulting model is utilized to compensate the unwanted temperature effect during the measurement process as mentioned in Figure 4 and will be further illustrated in Figures 5 and 6.

**Temperature compensation**

**Figure 5** shows a flowchart **500** of a method for providing a temperature dependent RF response model (e.g. $H_T(f)$) which may be utilized to compensate (e.g. normalize) temperature effect as part of a measuring process such as RF measuring to characterize and obtain a sample (e.g. OUT) properties. In step **505** a plurality of RF signals are transmitted towards a substance, for example step **505** includes transmitting an EM to the substance(s) and receiving by one or more sensors such as the sensors **130** and **135** of **Figure 1** or antennas **310** of **Figure 3** the frequency response of an EM wave travelling through the OUT. In step **510** the OUT, preferably the same OUT, is repeatedly measured, for example 2, 3, 4, 5, 6, 7, 8, 9, 10 or more times for example by the temperature sensor **170** of Figure 1 in a time interval of several seconds or milliseconds.

In step **520** the RF signals reflected from the sample are obtained for example by the RFSMU and the impedance (e.g. phase and amplitude) of the received EM wave is measure and analyzed for example by a processor such as the processing unit 160. Additionally the OUT temperatures are obtained.

In step **530** a temperature and frequency model is provided for the obtained temperatures and RF signals.

In step **540** a polynomial order for each function (e.g. Eq 3-5 below) and the model parameters are selected (preferably not more than the number of temperature values at which measurements are taken), and in step **550** the model coefficient parameters, e.g. temperature dependence parameters polynomials $h_{amp}(T)$ and $h_{phase}(T)$, are found using for example Least Squares method or other methods as known in the art.

For example, the following method is provided to show the relationship between the frequency responses $H_{T_1}(f)$ and $H_{T_0}(f)$ at temperatures $T_1$ and $T_0$ respectively of the same sample. This will provide a temperature- frequency responses dependence model as described by Eq (1) and (2) below:

$$\angle H_{T_1}(f) = \angle H_{T_0}(f) + h_{phase}(T_1 - T_0)g_{phase}(f) \tag{1}$$

$$20\log_{10}|H_{T_1}(f)| = 20\log_{10}|H_{T_0}(f)| + h_{amp}(T_1 - T_0)g_{amp}(f) \tag{2}$$

Where:

$g_{phase}(f)$ and $g_{amp}(f)$ are functions of the frequency.

$h_{amp}(T)$ and $h_{phase}(T)$ are functions of the temperature difference $T_1 - T_0$.

Specifically, for milk sample, which contains mostly water, the temperature dependence of dielectric properties is dominated by the temperature dependence of water, which is described, for example, by functions $h_{amp}(T)$ and $h_{phase}(T)$. The geometry of the probe determines the dependence of amplitude and phase on the dielectric constant, which in turn depends on the temperature. In some cases, the EM waves propagating through the OUT may be non-plane waves/spherical waves and etc. It is noted that according to methods of the invention the measured amplitude and phase may estimate all the sample's characteristics.

Moreover, the frequency dependence functions $g_{phase}(f)$, $g_{amp}(f)$ and/or temperature-dependence functions $h_{amp}(T)$ and $h_{phase}(T)$ can be approximated by their Taylor series according to Eq (3), (4) and (5):

$$\text{Define } \Delta T = (T_1 - T_0) \tag{3}$$

$$\angle H_{T_1}(f) = \angle H_{T_0}(f) + (c_k \Delta T^k + c_{k-1}\Delta T^{k-1} + \cdots + c_0)(a_n f^n + a_{n-1}f^{n-1} + \cdots +$$

$$a_1 f + a_0) \tag{4}$$

$$20\log_{10}|H_{T_1}(f)| = 20\log_{10}|H_{T_0}(f)| + (d_r \Delta T^r + d_{r-1}\Delta T^{r-1} + \cdots + d_0)(b_m f^m +$$

$$b_{m-1}f^{m-1} + \cdots + b_1 f + b_0) \tag{5}$$

In step **550** the OUT temperatures and RF signals are processed by for example the processor unit to yield temperature dependent RF response model which comprises frequency dependence functions $g_{phase}(f)$, $g_{amp}(f)$ and temperature-dependence functions $h_{amp}(T)$ and $h_{phase}(T)$

In step **560**, the parameters of the model can be estimated using for example Least Squares methods or other estimation methods as known in the art on the same OUT at several different known temperatures.

### Estimation model

**Figure 6** shows a flowchart **600** of a method for providing an estimation model for characterizing a substance, for example to obtain the dielectric properties of the substance and (optionally) further measure the properties of a substance such as fat, lactose, protein etc.

In step **610** one or more RF signals (e.g. M spectral points) reflected from several (N) samples (e.g. OUT) with different known properties (such as fat, protein and lactose) and the samples temperatures are measured. The signals and temperatures may be obtained from a measurement system such as the systems **100** or **200** of **Figures 1 and 2**. The following steps (e.g. steps **620-695**) comprise a set computations for providing an estimator which may be further utilized to obtain the properties of a substance such as milk.

Generally, the computation process includes obtaining a linear relation between the temperature normalized frequency response and the OUT properties. The resulting linear relation is utilized to compute the OUT properties for unknown samples. For example, measuring the temperature normalized response for 50 milk samples with known fat percentages. Finding the linear relation between the frequency responses and the fat percentage (of the known 50 samples) and then using this relation for another milk sample with unknown fat percentage. As mentioned the OUT properties are obtained by measuring the RF signals reflected from the OUT and analyzing the correlation between the measured signal and the OUT property as will be explained in details hereinbelow:

Step **620** comprises arranging the RF signals (log amp or phase) of measurement responses for one or more samples of the substance, for example in the form of matrix R of dimension M X N where M relates to the object's data points and N to the number of samples representing all the received responses of the object. The responses may be time domain signals, phase responses, log amplitude response or any combination therein.

Step **630** comprises computing the average of each row in matrix R (which is learnt from the calibration measurements) and transforming the R matrix to provide a linear transformation for the matrix R. The linear transformation result for the matrix R reduces the dimension of the parameter space and extract most of the information out of matrix R. In some cases a PCA (Principal Component analysis) transformation is used, however other transformation methods as known in the art may be used. The average of each row of the matrix R may be denoted as column vector $\mu$. In step **640** each column of the matrix R is subtracted to provide a new matrix C. In step **650** transformation steps are initialized, the transformation steps includes performing SVD (Singular Value Decomposition) to the matrix C ([U,S,V] = SVD(C)) and get 3 matrices U,S,V and in step **660** a matrix $U\_s$ is created which comprises the first $k$ rows of the matrix U. In step **670** a new feature matrix is created $R_U = U_S R$

In step **680** a vector of know properties (such as fat) of the substance is arranged denoted for example as vector y.

In steps **680** and **690** the estimation model scaler and vector are computed.

The linear estimator is derived using for example least squares methods using temperature normalized frequency responses which are labeled with the correct properties (different estimator for fat, protein and lactose).

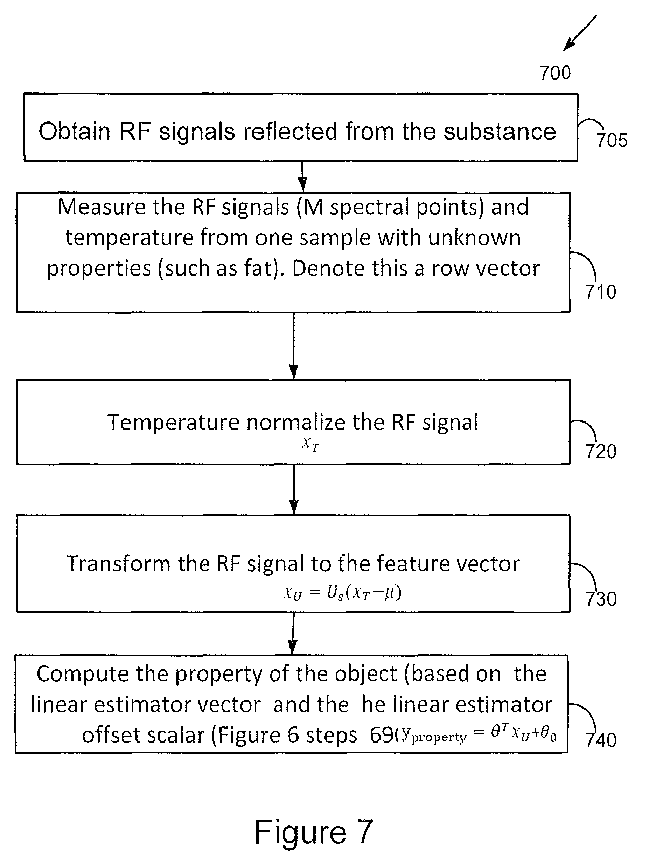

**Figure 7** shows a flowchart **700** of a method for characterizing a substance where the properties of the substance (e.g. OUT) are unknown. For example, identifying the content or properties of a mixture such as fruit mixture or milk or any liquid mixture by systems such as the systems of Figures 1-3, while compensating the temperature effect during the measurement process, in accordance with embodiments of the invention. The characterization method is based on the retrieved temperature dependent RF response model (as provided according the method illustrated in **Figure 5**) and the estimation model (as provided according the method illustrated in **Figure 6**). In step **705** multiple RF signals reflected from the substance are obtained from one or more RF sensors such as sensors 135, 130 of **Figure 1** or the antennas of Figure 3. In step **710** the obtained RF signals are measured, for example by the Radio Frequency Signal Measurement Unit (RFSMU). Additionally the substance's temperatures are measured, for example by a temperature processor. Alternatively, the substance temperatures may be obtained by measuring the RF signals reflected from the substance.

For example step **710** comprises processing the obtained multiple RF signals frequency response by a processor unit to measure the frequency response $H_i(f)$ at temperature $T_i$ and recording the temperature $T_i$ (e.g. the temperature in which the sample was measured) by the processor.

The processor may comprise a tangible medium embodying instructions, such as a computer readable memory embodying instructions of a computer program. Alternatively or in combination the processor may comprise logic such as gate array logic in order to perform one or more logic steps.

In step **720** the measured frequency responses, $H_{T_i}(f)$ measured at temperatures $T_i$ are transformed (e.g. normalized) to a frequency response at a reference temperature $T$ - $\hat{H}_i(f)$, where $\hat{H}_i(f)$ is defined as a normalized frequency response referenced temperature parameter. We also define $\Delta T = T - T_i$

The normalization step includes compensating (e.g. correcting) the temperature effect on the measured dielectric properties of the OUT according to the referenced temperature parameter to provide normalized frequency response of the OUT - $\hat{H}_i(f)$.

In some cases, $\hat{H}_i(f)$ is calculated according to the following Eq (6). Where the temperature dependent RF response model parameters $a_o$ till $a_k$, $b_0$ till $b_k$, $c_0$ till $c_k$ and $d_0$ till $d_k$ were computed in the **Figure 5**.

Eq (6)

$$\Delta T = (T_1 - T_0)$$

$$\angle H_{T_1}(f) = \angle H_{T_0}(f) + (c_k \Delta T^k + c_{k-1}\Delta T^{k-1} + \cdots + c_0)(a_n f^n + a_{n-1} f^{n-1} + \cdots + a_1 f + a_0)$$

$$20\log_{10}|H_{T_1}(f)| = 20\log_{10}|H_{T_0}(f)| + (d_r \Delta T^r + d_{r-1}\Delta T^{r-1} + \cdots + d_0)(b_m f^m + b_{m-1} f^{m-1} + \cdots + b_1 f + b_0)$$

In step 730 the RF signal Transformed to a feature vector

$$x_U = U_s(x_T - \mu)$$

Where the vector's parameters (e.g. **matrix $U_s$** and **vector $\mu$**) were calculated and provided according to the method illustrated in **Figure 6**.

In step **730** a linear estimator which includes processing the obtained multiple RF signals by the processor to measure the dielectric properties of the OUT based on the frequency response with the reference temperature is computed. Specifically, the linear estimator provides a model for characterizing the substance, e.g. the substance's properties, for example a milk's properties (fat, glucose etc.).

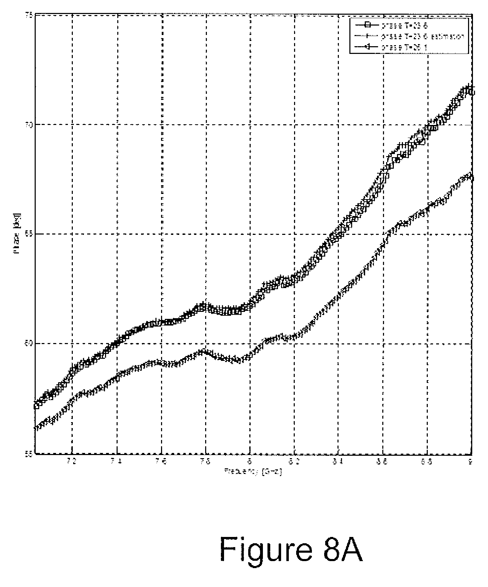

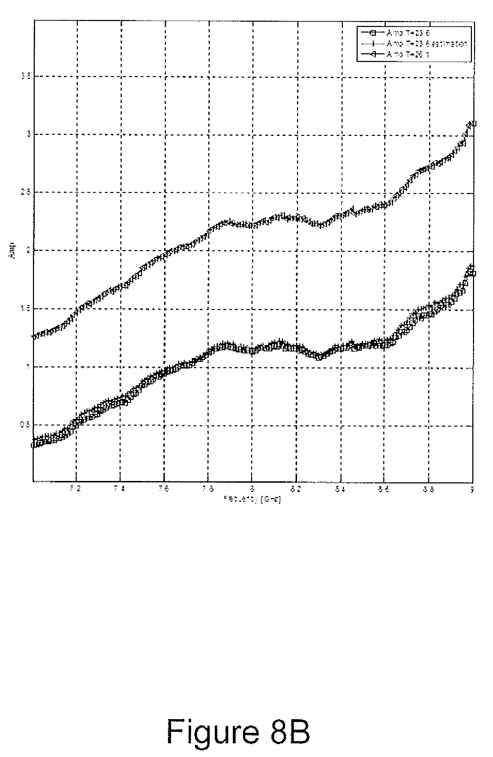

**Figures 8A** and **8B** show exemplary phase and amplitude responses of milk, suitable for incorporation in accordance with embodiments.

The phase and amplitude response of one milk sample at two different temperatures are shown to have characteristic features specific to the milk's fat content or percentage and temperature. Characteristic features include, for example, the general shape of the phase and amplitude response, the number of peaks and valleys in the spectra within a certain frequency range, and the corresponding frequencies or frequency ranges of said peaks and valleys of the phase and amplitude response. Based on such characteristic features, a characterization such as an RF system as described herein can determine the fat percentage or other parameters of the milk such as protein, lactose, somatics and urea (e.g., "3% fat", "5% lactose") of a sampled material, by comparing the measured phase and amplitude response data against the phase and amplitude response data of various materials stored in a universal database, or at the system database.

**Figure 8A** shows the phase response of the same milk sample at two different temperatures (e.g. 23.1 degrees and 26.1 degrees) and also the normalized phase response of the phase response at 26.1 degrees when normalized to a reference temperature 23.6 degrees (phase T=23.6 estimation). **Figure 8B** shows the amplitude response of the same milk sample at two different temperatures (denoted in the figure as 23.1 degrees and 26.1 degrees) and also the normalized amplitude response of the phase response at 26.1 degrees when normalized to a reference temperature 23.6 (denoted in the figure as Amp T=23.6 estimation) degrees.

**Figure 8A** and **8B** illustrate the resulted phase and amplitude responses following the normalization process for calibrating the temperature variation effect on the measured signal responses of the sample, in accordance with the present invention embodiments. As is clearly shown the phase responses for all ranges (e.g. 2-9 GHz) for the same sample are accordingly the same and the temperature effect is successfully eliminated.

In further embodiments, the processing unit may be a digital processing device including one or more hardware central processing units (CPU) that carry out the device's functions. In still further embodiments, the digital processing device further comprises an operating system configured to perform executable instructions. In some embodiments, the digital processing device is optionally connected a computer network. In further embodiments, the digital processing device is optionally connected to the Internet such that it accesses the World Wide Web. In still further embodiments, the digital processing device is optionally connected to a cloud computing infrastructure. In other embodiments, the digital processing device is optionally connected to an intranet. In other embodiments, the digital processing device is optionally connected to a data storage device.

In accordance with the description herein, suitable digital processing devices include, by way of non-limiting examples, server computers, desktop computers, laptop computers, notebook computers, sub-notebook computers, netbook computers, netpad computers, set-top computers, handheld computers, Internet appliances, mobile smartphones, tablet computers, personal digital assistants, video game consoles, and vehicles. Those of skill in the art will recognize that many smartphones are suitable for use in the system described herein. Those of skill in the art will also recognize that select televisions with optional computer network connectivity are suitable for use in the system described herein. Suitable tablet computers include those with booklet, slate, and convertible configurations, known to those of skill in the art.

In some embodiments, the digital processing device includes an operating system configured to perform executable instructions. The operating system is, for example, software, including programs and data, which manages the device's hardware and provides services for execution of applications. Those of skill in the art will recognize that suitable server operating systems include, by way of non-limiting examples, FreeBSD, OpenBSD, NetBSD®, Linux, Apple® Mac OS X Server®, Oracle® Solaris®, Windows Server®, and Novell® NetWare®. Those of skill in the art will recognize that suitable personal computer operating systems include, by way of non-limiting examples, Microsoft® Windows®, Apple® Mac OS X®, UNIX®, and UNIX-like operating systems such as GNU/Linux®. In some embodiments, the operating system is provided by cloud computing. Those of skill in the art will also recognize that suitable mobile smart phone operating systems include, by way of non-limiting examples, Nokia® Symbian® OS, Apple® iOS®, Research In Motion® BlackBerry OS®, Google® Android®, Microsoft® Windows Phone® OS, Microsoft® Windows Mobile® OS, Linux®, and Palm® WebOS®.

In some embodiments, the device includes a storage and/or memory device. The storage and/or memory device is one or more physical apparatuses used to store data or programs on a temporary or permanent basis. In some embodiments, the device is volatile memory and requires power to maintain stored information. In some embodiments, the device is non-volatile memory and retains stored information when the digital processing device is not powered. In further embodiments, the non-volatile memory comprises flash memory. In some embodiments, the non-volatile memory comprises dynamic random-access memory (DRAM). In some embodiments, the non-volatile memory comprises ferroelectric random access memory (FRAM). In some embodiments, the non-volatile memory comprises phase-change random access memory (PRAM). In other embodiments, the device is a storage device including, by way of non-limiting examples, CD-ROMs, DVDs, flash memory devices, magnetic disk drives, magnetic tapes drives, optical disk drives, and cloud computing based storage. In further embodiments, the storage and/or memory device is a combination of devices such as those disclosed herein.

In some embodiments, the digital processing device includes a display to send visual information to a user. In some embodiments, the display is a cathode ray tube (CRT). In some embodiments, the display is a liquid crystal display (LCD). In further embodiments, the display is a thin film transistor liquid crystal display (TFT-LCD). In some embodiments, the display is an organic light emitting diode (OLED) display. In various further embodiments, on OLED display is a passive-matrix OLED (PMOLED) or active-matrix OLED (AMOLED) display. In some embodiments, the display is a plasma display. In other embodiments, the display is a video projector. In still further embodiments, the display is a combination of devices such as those disclosed herein.

In some embodiments, the digital processing device includes an input device to receive information from a user. In some embodiments, the input device is a keyboard. In some embodiments, the input device is a pointing device including, by way of non-limiting examples, a mouse, trackball, track pad, joystick, game controller, or stylus. In some embodiments, the input device is a touch screen or a multi-touch screen. In other embodiments, the input device is a microphone to capture voice or other sound input. In other embodiments, the input device is a video camera to capture motion or visual input. In still further embodiments, the input device is a combination of devices such as those disclosed herein.

In some embodiments, the system disclosed herein includes one or more non-transitory computer readable storage media encoded with a program including instructions executable by the operating system of an optionally networked digital processing device. In further embodiments, a computer readable storage medium is a tangible component of a digital processing device. In still further embodiments, a computer readable storage medium is optionally removable from a digital processing device.

In some embodiments, a computer readable storage medium includes, by way of non-limiting examples, CD-ROMs, DVDs, flash memory devices, solid state memory, magnetic disk drives, magnetic tape drives, optical disk drives, cloud computing systems and services, and the like. In some cases, the program and instructions are permanently, substantially permanently, semi-permanently, or non-transitorily encoded on the media. In some embodiments, the system disclosed herein includes at least one computer program, or use of the same. A computer program includes a sequence of instructions, executable in the digital processing device's CPU, written to perform a specified task. Computer readable instructions may be implemented as program modules, such as functions, objects, Application Programming Interfaces (APIs), data structures, and the like, that perform particular tasks or implement particular abstract data types. In light of the disclosure provided herein, those of skill in the art will recognize that a computer program may be written in various versions of various languages.

The functionality of the computer readable instructions may be combined or distributed as desired in various environments. In some embodiments, a computer program comprises one sequence of instructions. In some embodiments, a computer program comprises a plurality of sequences of instructions. In some embodiments, a computer program is provided from one location. In other embodiments, a computer program is provided from a plurality of locations. In various embodiments, a computer program includes one or more software modules. In various embodiments, a computer program includes, in part or in whole, one or more web applications, one or more mobile applications, one or more standalone applications, one or more web browser plug-ins, extensions, add-ins, or add-ons, or combinations thereof.

In some embodiments, a computer program includes a mobile application provided to a mobile digital processing device. In some embodiments, the mobile application is provided to a mobile digital processing device at the time it is manufactured. In other embodiments, the mobile application is provided to a mobile digital processing device via the computer network described herein.

In view of the disclosure provided herein, a mobile application is created by techniques known to those of skill in the art using hardware, languages, and development environments known to the art. Those of skill in the art will recognize that mobile applications are written in several languages. Suitable programming languages include, by way of non-limiting examples, C, C++, C#, Objective-C, Java™, Javascript, Pascal, Object Pascal, Python™, Ruby, VB.NET, WML, and XHTML/HTML with or without CSS, or combinations thereof.

Suitable mobile application development environments are available from several sources. Commercially available development environments include, by way of non-limiting examples, AirplaySDK, alcheMo, Appcelerator®, Celsius, Bedrock, Flash Lite, .NET Compact Framework, Rhomobile, and WorkLight Mobile Platform. Other development environments are available without cost including, by way of non-limiting examples, Lazarus, MobiFlex, MoSync, and Phonegap. Also, mobile device manufacturers distribute software developer kits including, by way of non-limiting examples, iPhone and iPad (iOS) SDK, Android™ SDK, BlackBemy® SDK, BREW SDK, Palm® OS SDK, Symbian SDK, webOS SDK, and Windows® Mobile SDK.

Those of skill in the art will recognize that several commercial forums are available for distribution of mobile applications including, by way of non-limiting examples, Apple® App Store, Android™ Market, BlackBemy® App World, App Store for Palm devices, App Catalog for webOS, Windows® Marketplace for Mobile, Ovi Store for Nokia® devices, Samsung® Apps, and Nintendo® DSi Shop.

In some embodiments, the system disclosed herein includes software, server, and/or database modules, or use of the same. In view of the disclosure provided herein, software modules are created by techniques known to those of skill in the art using machines, software, and languages known to the art. The software modules disclosed herein are implemented in a multitude of ways. In various embodiments, a software module comprises a file, a section of code, a programming object, a programming structure, or combinations thereof. In further various embodiments, a software module comprises a plurality of files, a plurality of sections of code, a plurality of programming objects, a plurality of programming structures, or combinations thereof. In various embodiments, the one or more software modules comprise, by way of non-limiting examples, a web application, a mobile application, and a standalone application. In some embodiments, software modules are in one computer program or application. In other embodiments, software modules are in more than one computer program or application. In some embodiments, software modules are hosted on one machine. In other embodiments, software modules are hosted on more than one machine. In further embodiments, software modules are hosted on cloud computing platforms. In some embodiments, software modules are hosted on one or more machines in one location. In other embodiments, software modules are hosted on one or more machines in more than one location.

In some embodiments, the system disclosed herein includes one or more databases, or use of the same. In view of the disclosure provided herein, those of skill in the art will recognize that many databases are suitable for storage and retrieval of information as described herein. In various embodiments, suitable databases include, by way of non-limiting examples, relational databases, non-relational databases, object oriented databases, object databases, entity-relationship model databases, associative databases, and XML databases. In some embodiments, a database is internet-based. In further embodiments, a database is web-based. In still further embodiments, a database is cloud computing-based. In other embodiments, a database is based on one or more local computer storage devices.

In the above description, an embodiment is an example or implementation of the inventions. The various appearances of "one embodiment," "an embodiment" or "some embodiments" do not necessarily all refer to the same embodiments.

Although various features of the invention may be described in the context of a single embodiment, the features may also be provided separately or in any suitable combination. Conversely, although the invention may be described herein in the context of separate embodiments for clarity, the invention may also be implemented in a single embodiment.

Reference in the specification to "some embodiments", "an embodiment", "one embodiment" or "other embodiments" means that a particular feature, structure, or characteristic described in connection with the embodiments is included in at least some embodiments, but not necessarily all embodiments, of the inventions.

It is to be understood that the phraseology and terminology employed herein is not to be construed as limiting and are for descriptive purpose only.

The principles and uses of the teachings of the present invention may be better understood with reference to the accompanying description, figures and examples.

It is to be understood that the details set forth herein do not construe a limitation to an application of the invention.

Furthermore, it is to be understood that the invention can be carried out or practiced in various ways and that the invention can be implemented in embodiments other than the ones outlined in the description above.

It is to be understood that the terms "including", "comprising", "consisting" and grammatical variants thereof do not preclude the addition of one or more components, features, steps, or integers or groups thereof and that the terms are to be construed as specifying components, features, steps or integers.

If the specification or claims refer to "an additional" element, that does not preclude there being more than one of the additional element.

It is to be understood that where the claims or specification refer to "a" or "an" element, such reference is not be construed that there is only one of that element.

It is to be understood that where the specification states that a component, feature, structure, or characteristic "may", "might", "can" or "could" be included, that particular component, feature, structure, or characteristic is not required to be included.

Where applicable, although state diagrams, flow diagrams or both may be used to describe embodiments, the invention is not limited to those diagrams or to the corresponding descriptions. For example, flow need not move through each illustrated box or state, or in exactly the same order as illustrated and described.

Methods of the present invention may be implemented by performing or completing manually, automatically, or a combination thereof, selected steps or tasks.

The descriptions, examples, methods and materials presented in the claims and the specification are not to be construed as limiting but rather as illustrative only.

Meanings of technical and scientific terms used herein are to be commonly understood as by one of ordinary skill in the art to which the invention belongs, unless otherwise defined.

The present invention may be implemented in the testing or practice with methods and materials equivalent or similar to those described herein.

While the invention has been described with respect to a limited number of embodiments, these should not be construed as limitations on the scope of the invention, but rather as exemplifications of some of the preferred embodiments. Other possible variations, modifications, and applications are also within the scope of the invention. Accordingly, the scope of the invention should not be limited by what has thus far been described, but by the appended claims and their legal equivalents.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

**Claims**

1. A system for characterizing a substance, said system comprising:

    a housing body having a cavity therein wherein said cavity is configured to contain said substance;
    a transmit unit configured to transmit a plurality of Radio Frequency (RF) signals towards said substance;
    at least one electromagnetic sensor, wherein said at least one electromagnetic sensor is configured to receive RF signals affected by said substance and provide RF responses data of said substance;
    a Radio Frequency Signals Measurement Unit (RFSMU) configured to receive said RF responses data and measure said RF responses data;
    a temperature sensor configured to measure the temperatures of said substance over time; and
    at least one processing unit, said at least one processing unit is configured to:

        retrieve temperature dependent RF response model of said substance;
        receive the temperatures and the measured RF responses data of said substance;

normalize the measured RF responses of said substance according to said temperature dependent RF response model to generate compensated measured RF responses of said substance at a reference temperature; and

process said compensated measured RF responses to identify the characteristics of said substance.

2. The system of claim 1 wherein said characteristics are dielectric properties of said substance.

3. The system of claim 1 wherein said temperature dependent RF response model is configured by measuring said RF responses data for several values of temperatures and fitting said model.

4. The system of claim 1 wherein said substance is a mixture of materials and the characteristics are percentage values of the mixture of materials.

5. The system of claim 1 wherein the identification of characteristics of said substance is performed using a model generated by measuring said compensated RF responses for several values of said characteristics and fitting said model.

6. The system of claim 1 wherein the temperatures are obtained by the at least one electromagnetic sensor.

7. The system of claim 1 wherein said at least one electromagnetic sensor comprises at least two antennas and wherein said at least one processing unit is configured to analyze a passage of energy transmitted from at least one of said antennas to at least one other antenna.

8. The system of claim 1 wherein said at least one electromagnetic sensor is a capacitive sensor and wherein said sensor is configured to spatially resolve the dielectric properties of the said substance.

9. The system of claim 1 wherein said one electromagnetic sensor is attached internally or externally to the housing body.

10. The system of claim 1 wherein said at least one electromagnetic sensor is connected to said housing body and said transmit unit.

11. The system of claim 1, wherein said at least one electromagnetic sensor in not in direct contact with said substance.

12. The system of claim 1 wherein said substance is a non-solid substance.

13. The system of claim 1 wherein said substance is in the form selected from a group consisting of: liquid, paste, gas, granular material.

14. The system of claim 1 wherein said substance is an agricultural product.

15. The system of claim 2 wherein the dielectric properties of the substance are configured based on an estimation model, said estimation model is based on measuring RF signals affected by plurality of substances, wherein said plurality of substances parameters are known.

Figure 1

Figure 2A

280

260

210

170

220

# Figure 2B

Figure 3

Generating a plurality of RF signals towards a substance

400

410

Collecting multiple RF signals from the substance

420

Measuring the substance temperature

430

Processing the reflected multiple RF signals to yield a temperature dependent RF response model

440

normalizingthe RF responses of the substance according to the temperature dependent RF response model to generate compensated RF responses of said substance at a reference temperature

450

Processing the normalized RF signals to obtain the dielectric properties of the substance

460

Displaying the properties of the substance

470

Figure 4

```
┌─────────────────────────────────────┐
│  Generating a plurality of RF signals towards a │
│            substance                 │
└─────────────────────────────────────┘  505
                  │
                  ▼
┌─────────────────────────────────────┐
│  Measure the substance's temperature over │
│               time                   │
└─────────────────────────────────────┘  510
                  │
                  ▼
┌─────────────────────────────────────┐
│     Obtain RF signals reflected from the │
│  substance and temperature and measure the │
│          reflected RF signals        │
└─────────────────────────────────────┘  520
                  │
                  ▼
┌─────────────────────────────────────┐
│  Provide temperature and frequency model for │
│   the obtained temperatures and RF signals │
└─────────────────────────────────────┘  530
                  │
                  ▼
┌─────────────────────────────────────┐
│   Select the order of the model's functions and │
│             parameters               │
└─────────────────────────────────────┘  540
                  │
                  ▼
┌─────────────────────────────────────┐
│  Process the substance temperatures and RF │
│  signals to yield a  temperature frequency/ │
│         phase dependant  model       │
└─────────────────────────────────────┘  550
                  │
                  ▼
┌─────────────────────────────────────┐
│       Estimate the model  parameters │
│   using for example  Least Squares fit │
└─────────────────────────────────────┘  560
```

500

Figure 5

Figure 6

700

Obtain RF signals reflected from the substance — 705

Measure the RF signals (M spectral points) and temperature from one sample with unknown properties (such as fat). Denote this a row vector — 710

Temperature normalize the RF signal
$x_T$ — 720

Transform the RF signal to the feature vector
$x_U = U_s(x_T - \mu)$ — 730

Compute the property of the object (based on the linear estimator vector and the he linear estimator offset scalar (Figure 6 steps 69($y_{\text{property}} = \theta^T x_U + \theta_0$ — 740

Figure 7

# Figure 8A

Figure 8B

Europäisches
Patentamt

European
Patent Office

Office européen
des brevets

# EUROPEAN SEARCH REPORT

Application Number

EP 16 16 6198

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/015792 A1 (SCOTT BENTLEY N [US]) 17 January 2008 (2008-01-17) * paragraphs [0002], [0034] - [0044], [0069], [0070] * * figures 1-5 * | 1-5,9, 12,13,15 | INV. G01N33/06 G01N22/00 |
| X | KING R J: "MICROWAVE SENSORS FOR PROCESS CONTROL PART I: TRANSMISSION SENSORS", SENSORS, NORTH AMERICAN TECHNOLOGY, PETERBOROUGH, NH, US, 1 September 1992 (1992-09-01), pages 68-74, XP002063123, ISSN: 0746-9462 | 1-5,7, 9-15 | |
| Y | * page 68, left-hand column, paragraph 1 - right-hand column, paragraph 3 * * page 69, left-hand column, paragraph 1 - page 73, left-hand column, paragraph 2 * * figures 1,2,5 * | 6 | |
| X | WO 2004/023125 A2 (PENDRAGON MEDICAL LTD [CH]; CADUFF ANDREAS [CH]; HIRT ETIENNE [CH]; SC) 18 March 2004 (2004-03-18) * page 1, lines 5-11 * * page 5, line 30 - page 9, line 3 * * page 11, line 12 - page 12, line 27 * * figures 1-3,9,10 * | 1,2,4, 8-14 | |
| Y | US 2007/188372 A1 (LEATH SHANE R [NZ]) 16 August 2007 (2007-08-16) * paragraphs [0001], [0002], [0076] - [0091] * * figure 1 * | 6 | |

TECHNICAL FIELDS
SEARCHED      (IPC)

G01N

The present search report has been drawn up for all claims

| Place of search | Date of completion of the search | Examiner |
|---|---|---|
| The Hague | 16 September 2016 | Couteau, Olivier |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

                                                            
& : member of the same patent family, corresponding
   document

EPO FORM 1503 03.82 (P04C01)

2

## ANNEX TO THE EUROPEAN SEARCH REPORT
## ON EUROPEAN PATENT APPLICATION NO.

EP 16 16 6198

This annex lists the patent family members relating to the patent documents cited in the above-mentioned European search report.
The members are as contained in the European Patent Office EDP file on
The European Patent Office is in no way liable for these particulars which are merely given for the purpose of information.

16-09-2016

| Patent document cited in search report | | | Publication date | Patent family member(s) | | | Publication date |
|---|---|---|---|---|---|---|---|
| US 2008015792 | A1 | | 17-01-2008 | CA<br>GB<br>US<br>WO | 2655407<br>2454107<br>2008015792<br>2007146357 | A1<br>A<br>A1<br>A2 | 21-12-2007<br>29-04-2009<br>17-01-2008<br>21-12-2007 |
| WO 2004023125 | A2 | | 18-03-2004 | AU<br>WO | 2003264797<br>2004023125 | A1<br>A2 | 29-03-2004<br>18-03-2004 |
| US 2007188372 | A1 | | 16-08-2007 | AU<br>BR<br>CA<br>EP<br>NZ<br>US<br>WO | 2005272237<br>PI0514290<br>2576996<br>1776580<br>534673<br>2007188372<br>2006016824 | A1<br>A<br>A1<br>A1<br>A<br>A1<br>A1 | 16-02-2006<br>10-06-2008<br>16-02-2006<br>25-04-2007<br>31-03-2006<br>16-08-2007<br>16-02-2006 |

EPO FORM P0459

For more details about this annex : see Official Journal of the European Patent Office, No. 12/82

**EP 3 086 118 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- IL 2015050126 W
- US 20060255276 A